# EUROPEAN PATENT APPLICATION

(11) **EP 3 029 934 A1**
(43) Date of publication of application: **08.06.2016**
(21) Application number: 14854635.1
(22) Date of filing: 15.10.2014
(51) Int. Cl.: H04N 13/04, A61B 1/04, H04N 13/02

(54) **IMAGE OUTPUTTING DEVICE**

(30) Priority: 18.10.2013 JP 2013217376
(71) Applicant: OLYMPUS CORPORATION, Shibuya-ku Tokyo 151-0072 (JP)
(72) Inventor: ASATORI Sachiko, Tokyo 151-0072 (JP); ITO Takehiko, Tokyo 151-0072 (JP)
(74) Representative: Gunzelmann, Rainer
(86) International application number: PCT/JP2014/077412
(87) International publication number: WO 2015/056701

(57) **Abstract**

An image output apparatus includes: a stereoscopic image generation unit that is provided with a first image and a second image with a parallax relative to the first image and that generates a stereoscopic image based on the first image and the second image, wherein the first image is provided from a first image acquisition unit that acquires the first image by picking up an image of a subject, and the second image is provided from a second image acquisition unit that acquires the second image by picking up an image of the subject; a two-dimensional image generation unit that generates a two-dimensional image based on the first image or the second image; a plurality of output units that can output the stereoscopic image and the two-dimensional image; and a switching unit that can switch, for each of the output units, which image of the stereoscopic image and the two-dimensional image is supplied to the plurality of output units.

## Description

### Technical Field

The present invention relates to an image output apparatus that can output a 3D image.

### Background Art

In recent years, a medical observation apparatus, such as an endoscope and a surgical microscope, for observing an operation region in a medical action, such as a surgery, is widely used. Furthermore, a manipulation using a medical stereoscopic observation system, such as a stereoscopic (3D) endoscope, that can stereoscopically display an observed image is becoming prevalent, particularly in a field of surgery. For example, an attempt is made in a surgery to display a tomographic image or the like of an affected part acquired before the surgery, on top of a 3D image of the affected part that is actually picked up.

The 3D image can display a depth, and the 3D image is effective in a surgery or the like. However, the 3D image is obtained by a left eye image and a right eye image with a parallax, and an amount of data is larger than a normal two-dimensional (2D) image. Therefore, a necessary recording capacity is large when the 3D image is used in recording of a case or the like, and there is also a drawback that power consumption in an image output unit is large.

Accordingly, Japanese Patent Application No. 6-254046 discloses an apparatus that can switch whether to output a 2D image or to output a 3D image as an image output. In the apparatus of Japanese Patent Application No. 6-254046, the 3D image is not outputted when the 2D image is outputted. In this case, the power consumption can be decreased, and the necessary recording capacity can be reduced.

By the way, an image to be outputted can be used at various output destinations. For example, the outputted image can be used in a monitor for surgeon, a monitor for medical staff, a monitor for subject, and the like in an operating room, and the outputted image can also be used for recording. Therefore, an image output apparatus that includes a plurality of output ports to output the outputted image to a plurality of devices is also widely used.

However, which one of the 2D image and the 3D image is necessary is different in each device that uses the outputted image. For example, the 3D image can be provided to the monitor for surgeon to facilitate the observation, and the 2D image can be provided to a recording device from the viewpoint of the capacity. Some of surgeons who view the observed image displayed on a same monitor cannot recognize a depth from the 3D image and cannot correctly recognize the image unless the image is a 2D image. Which one of the 2D image and the 3D image is necessary may vary even within a same device.

However, the apparatus of Japanese Patent Application No. 6-254046 cannot output the 3D image while the 2D image is outputted and cannot output the 2D image while the 3D image is outputted. Therefore, the 2D image is outputted or the 3D image is outputted from all output ports, and there is a problem that an image suitable for a used device cannot be outputted.

An object of the present invention is to provide an image output apparatus that can selectively output a 2D image or a 3D image in each port of a plurality of output ports.

### Disclosure of Invention

### Means for Solving the Problem

The present invention provides an image output apparatus including: a stereoscopic image generation unit that is provided with a first image and a second image with a parallax relative to the first image and that generates a stereoscopic image based on the first image and the second image, wherein the first image is provided from a first image acquisition unit that acquires the first image by picking up an image of a subject, and the second image is provided from a second image acquisition unit that acquires the second image by picking up an image of the subject; a two-dimensional image generation unit that generates a two-dimensional image based on the first image or the second image; a plurality of output units that can output the stereoscopic image and the two-dimensional image; and a switching unit that can switch, for each of the output units, which image of the stereoscopic image and the two-dimensional image is supplied to the plurality of output units.

### Brief Description of the Drawings

Fig. 1 is a block diagram showing an image output apparatus according to a first embodiment of the present invention;
Fig. 2 is a block diagram showing a modification of the first embodiment;
Fig. 3 is a block diagram showing a second embodiment;
Fig. 4 is a flowchart for describing operation of the second embodiment; and
Fig. 5 is a block diagram showing an endoscope system that can independently set parameters for left eye and for right eye.

### Best Mode for Carrying Out the Invention

Hereinafter, embodiments of the present invention will be described in detail with reference to the drawings.

### (First Embodiment)

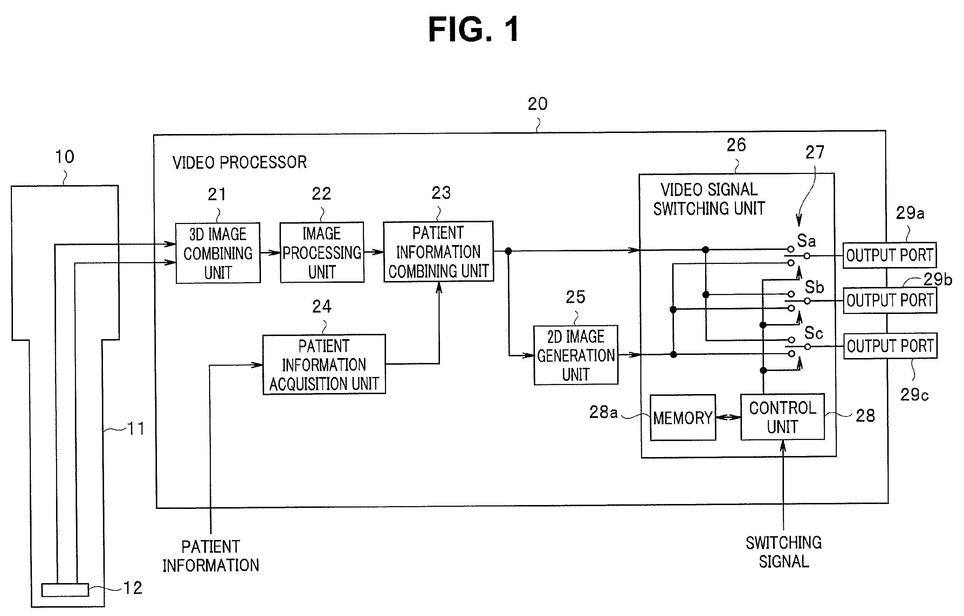

Fig. 1 is a block diagram showing an image output apparatus according to a first embodiment of the present invention. In the present embodiment, the image output apparatus is applied to an endoscope system including an endoscope and a video processor.

The endoscope system of Fig. 1 includes an endoscope 10 and a video processor 20. On a distal end side, the endoscope 10 includes an elongated insertion portion 11 that can be inserted to a lumen and the like, and a proximal end side is detachably connected to the video processor 20 through a connector or the like not shown.

An image pickup unit 12 that picks up images of a video of an object in a lumen or the like is disposed on a distal end of the insertion portion 11. An illumination lens not shown is disposed on the distal end of the insertion portion 11, and illuminating light is applied to the object through the illumination lens. The image pickup unit 12 includes a CCD, a CMOS sensor, or the like, and return light from the object enters an image pickup surface. The image pickup unit 12 photoelectrically converts entered object optical images and sequentially outputs image pickup outputs based on accumulated charge.

In the present embodiment, the image pickup unit 12 is configured to output parallax images for generating a 3D image. For example, the image pickup unit 12 may include a pair of image pickup lenses for right eye and for left eye and may acquire picked-up images for right eye and for left eye from object optical images entered from respective lenses. The image pickup unit 12 may also be configured to obtain a 3D image by dividing a region of one lens without using twin image pickup lenses. Although one image pickup unit 12 outputs the picked-up images for left eye and for right eye in the illustrated example, two image pickup units may output the picked-up images for left eye and for right eye.

The picked-up images for left eye and for right eye from the image pickup unit 12 are supplied to the video processor 20. A 3D image synthesis unit 21 of the video processor 20 synthesizes the picked-up images for left eye and for right eye from the image pickup unit 12 and outputs a 3D image to an image processing unit 22. The image processing unit 22 applies predetermined image signal processing, such as various image signal processings including white balance processing, edge enhancement processing, and enlargement/reduction processing, to the inputted 3D image and then outputs the 3D image to a patient information synthesis unit 23. The image processing unit 22 can output the 3D image in a standard according to a standard of a device at an output destination of observed images. For example, the image processing unit 22 outputs a 3D image in a 3G-SDI format.

An operator can input patient information, such as patient name and patient ID, through an input apparatus not shown, and a patient information acquisition unit 24 is configured to acquire the patient information and output the patient information to the patient information synthesis unit 23. The patient information synthesis unit 23 superimposes the inputted patient information on the 3D image and outputs the 3D image. The 3D image from the patient information synthesis unit 23 is provided to a video signal switching unit 26 and a 2D image generation unit 25.

The 2D image generation unit 25 generates a 2D image from the 3D image. For example, the 2D image generation unit 25 may generate the 2D image based on the left image or the right image included in the 3D image. The 2D image generation unit 25 is configured to output the generated 2D image to the video signal switching unit 26. Note that the 2D image generation unit 25 can output the 2D image in a standard according to a standard of a device at an output destination of observed images. For example, the 2D image generation unit 25 outputs a 2D image in an HD-SDI format.

The video signal switching unit 26 includes a selector 27, a control unit 28, and a memory 28a. The selector 27 includes three switching units Sa to Sc, and each of the switching units Sa to Sc includes two input ends. The 3D image from the patient information synthesis unit 23 is supplied to one of the input ends of each of the switching units Sa to Sc, and the 2D image from the 2D image generation unit 25 is supplied to the other input end of each of the switching units Sa to Sc. The control unit 28 separately controls each of the switching units Sa to Sc, and the switching units Sa to Sc are configured to select one of the two inputs and to output the image to output ports 29a to 29c, respectively.

A switching signal based on operation of the operator is inputted to the control unit 28. The operator can use the input apparatus not shown to perform operation of selecting which image of the 3D image and the 2D image is to be outputted, for each of the output ports 29a to 29c. The switching signal based on the operation is supplied from the input apparatus to the control unit 28. The control unit 28 stores information based on the switching signal in the memory 28a and generates a switch control signal based on the switching signal to separately control each of the switching units Sa to Sc of the selector 27 to output one of the two inputs according to the user operation.

Note that although the selector 27 outputs image outputs of three systems through the switching units of three systems in the example illustrated in Fig. 1, the number of switching units can be appropriately set, and it is apparent that image outputs of two systems or four or more systems can be outputted.

The output ports 29a to 29c are formed by a standard corresponding to a device at an output destination not shown and can supply a 2D or 3D image from each of the switching units Sa to Sc of the video signal switching unit 16 to the device at the output destination. In this way, the device at the output destination can display and record a 3D endoscopic image or a 2D endoscopic image.

Next, operation of the embodiment with the configuration will be described.

When observation by the endoscope 10 is started, the image pickup unit 12 photoelectrically converts an object image and outputs picked-up images for left eye and for right eye. The picked-up images for left eye and for right eye are provided to the 3D image synthesis unit 21 of the video processor 20. The 3D image synthesis unit 21 synthesizes the inputted picked-up images for left eye and for right eye to obtain a 3D image. The 3D image is supplied to the image processing unit 22, and predetermined image signal processing is applied. A 3D image that can be used in the device at the output destination is obtained.

The patient information acquisition unit 24 acquires patient information and outputs the patient information to the patient information synthesis unit 23. The patient information synthesis unit 23 superimposes the patient information, such as patient name, on the inputted 3D image and outputs the 3D image. The 3D image from the patient information synthesis unit 23 is supplied to the video signal switching unit 26 and is also supplied to the 2D image generation unit 25. The 2D image generation unit 25 converts the inputted 3D image to a 2D image that can be used in the device at the output destination and outputs the 2D image to the video signal switching unit 26.

The 3D image from the patient information synthesis unit 23 and the 2D image from the 2D image generation unit 25 are inputted to each of the switching units Sa to Sc included in the selector 27 of the video signal switching unit 26. Each of the switching units Sa to Sc is separately controlled based on a switch control signal from the control unit 28 and selectively outputs one of the two inputs.

A switching signal based on the user operation is inputted to the control unit 28 of the video signal switching unit 26. The control unit 28 generates a switch control signal for separately controlling the switching units Sa to Sc based on the switching signal. Now, it is assumed that a switching signal for outputting a 3D image from the output ports 29a and 29b and outputting a 2D image from the output port 29c is generated by user operation, for example. Based on the switching signal, the control unit 28 causes the switching units Sa and Sb to select the 3D image from the patient information synthesis unit 23 and causes the switching unit Sc to select the 2D image from the 2D image generation unit 25.

In this way, the switching units Sa to Sc separately select and output the 3D image or the 2D image according to the user operation. The output ports 29a to 29c output the images respectively supplied from the switching units Sa to Sc to devices at output destinations not shown.

For example, it is assumed that the output ports 29a and 29b are connected to monitors for surgeon and for medical staff and that the output port 29c is connected to a recording device. Through user operation, the operator generates a switching signal for outputting a 3D image from the output ports 29a and 29b and for outputting a 2D image from the output port 29c. The 3D image is supplied to the monitors for surgeon and for medical staff, and the 2D image is supplied to the recording device.

In this case, a surgeon and the like can view the 3D image with a feeling of depth on the monitors for surgeon and for medical staff, and this is significantly effective for a surgery and the like. Furthermore, the 2D image with a relatively small amount of data is provided to the recording device, and a recording capacity necessary for the recording device can be reduced.

Furthermore, even if there is a surgeon who can recognize only 2D images, two monitors can be prepared, and the two monitors can supply the 2D image and the 3D image, respectively. The 3D image can be displayed for a surgeon who feels that 3D images are easier to view, and the 2D image can be displayed for a surgeon who feels that 2D images are easier to view.

Note that although the example of generating the switching signal based on the user operation is described, if which one of the 3D image and the 2D image is to be outputted from the output ports is determined in advance, this information may be stored in advance in the memory 28a, and the control unit 28 may separately control the switching units Sa to Sc based on the information.

In this way, a plurality of output ports are arranged, and the switching units that separately switch the 3D image and the 2D image to output the image to each output port are arranged in the present embodiment. Therefore, the 3D image or the 2D image can be selectively outputted to each output port. As a result, the 3D image can be provided to a device in which the 3D image is suitable, and the 2D image can be supplied to a device in which the 2D image is suitable. This is highly convenient.

### (Modification)

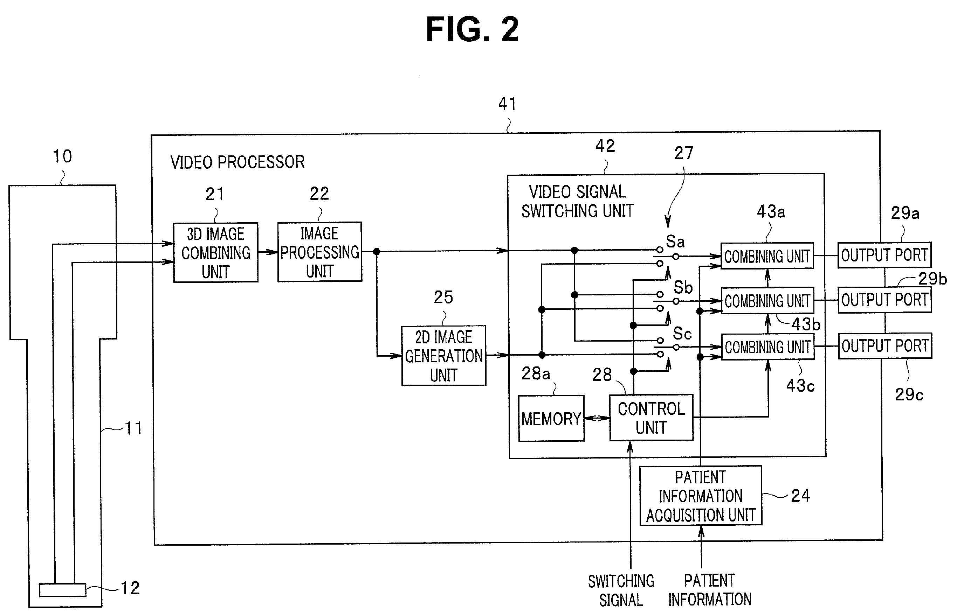

Fig. 2 is a block diagram showing a modification of the first embodiment. In Fig. 2, the same constituent elements as in Fig. 1 are designated with the same signs, and the description will not be repeated. In the image output apparatus of Fig. 1, the patient information is also superimposed in the 3D image and the 2D image. The patient information is displayed not only in the image displayed on the monitor, but the patient information is also superimposed in the 3D image or the 2D image and recorded in the recording device. The recording may not be preferable from the viewpoint of protection of personal information.

Therefore, whether to output the 3D image or the 2D image provided with the patient information or to output the 3D image or the 2D image not provided with the patient information can be switched for each output port in the present modification. A video processor 41 according to the present modification is different from the video processor 20 of Fig. 1 in that the patient information synthesis unit 23 is excluded and that a video signal switching unit 42 is adopted in place of the video signal switching unit 26. The 3D image from the image processing unit 22 is supplied to the video signal switching unit 42 and the 2D image generation unit 25.

The video signal switching unit 42 includes synthesis units 43a to 43c. The outputs of the switching units Sa to Sc included in the selector 27 are supplied to the synthesis units 43a to 43c, respectively. The synthesis units 43a to 43c are provided with the patient information from the patient information acquisition unit 24.

A switching signal is inputted to the control unit 28. The switching signal is for setting selection of one of the 3D image and the 2D image to be inputted to the switching units Sa to Sc based on user operation. Furthermore, in the present modification, a user can use an input apparatus not shown to set the output port for which the patient information is superimposed on a video signal to be outputted, and this setting information is provided as the switching signal to the control unit 28.

The control unit 28 stores information based on the switching signal in the memory 28a. The control unit 28 can control the switching units Sa to Sc based on the information stored in the memory 28a to switch whether to output the 3D image or to output the 2D image to each of the output ports 29a to 29c. Furthermore, in the present modification, the control unit 28 can control synthesis processes of the synthesis units 43a to 43c based on the information stored in the memory 28a and can permit only the synthesis unit among the synthesis units 43a to 43c designated by the switching signal to execute the synthesis process of the patient information.

In the modification with the configuration, the image to be outputted can be separately switched between the 3D image and the 2D image for each port of the output ports 29a to 29c as in the first embodiment. Furthermore, the user can set whether to superimpose the patient information on the image to be outputted from each of the output ports 29a to 29c in the present modification.

For example, it is assumed that the user performs setting operation for superimposing the patient information only on the image of the output port 29b. A switching signal based on the setting operation is supplied to the control unit 28, and the control unit 28 controls the synthesis units 43a to 43c to permit the synthesis process of the synthesis unit 43b and to prohibit the synthesis processes of the synthesis units 43a and 43c. As a result, the patient information is superimposed only on the 3D image or the 2D image supplied to the synthesis unit 43b through the switching unit Sb, and the image is outputted to the output port 29b. The patient information is not superimposed on the 3D image or the 2D image supplied to the synthesis units 43a and 43c through the switching units Sa and Sc, respectively, and the image is outputted to the output ports 29a and 29c.

In this way, whether to superimpose the patient information on the image outputted from each output port can be separately set for each output port in the present modification. As a result, for example, it is possible not to superimpose the patient information on the image outputted from the output port connected to the recording device, and this is useful from the viewpoint of the protection of personal information.

Note that in the present modification, whether to superimpose the patient information can also be determined in advance for each port, and this information can be stored in the memory 28a. This makes the setting operation of the user unnecessary, and the image provided with the patient information can be outputted only from the output port determined in advance.

### (Second Embodiment)

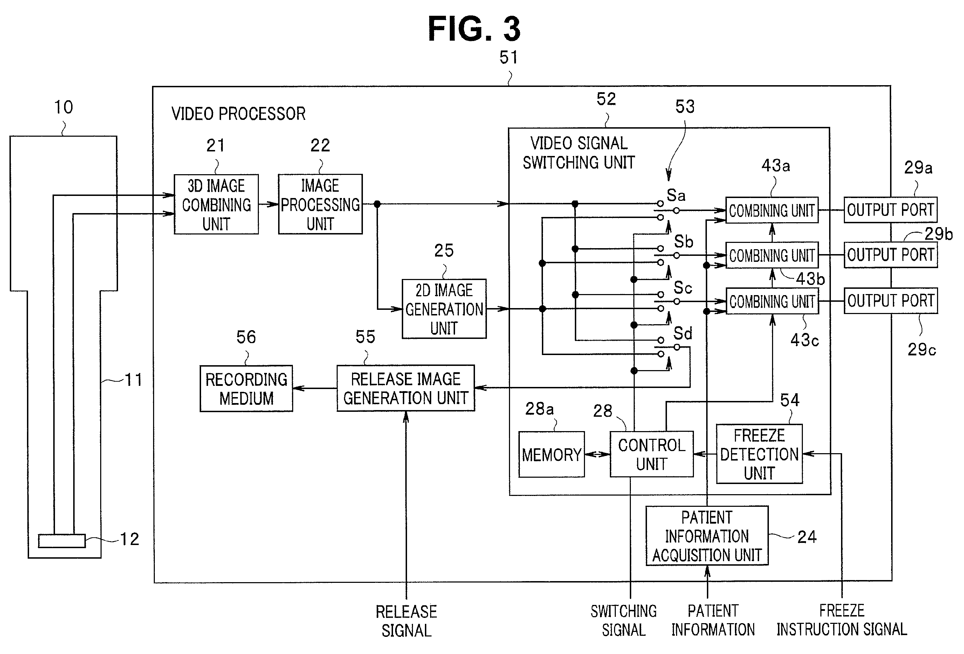

Fig. 3 is a block diagram showing a second embodiment. In Fig. 3, the same constituent elements as in Fig. 2 are designated with the same signs, and the description will not be repeated.

In the first embodiment, the 3D image and the 2D image can be separately selected for each port as an image to be outputted from the output port. In the present embodiment, the 3D image or the 2D image can be further selected as a release image (still image) to be recorded when there is release operation.

A video processor 51 according to the present embodiment is different from the video processor 41 of Fig. 2 in that a video signal switching unit 52 is adopted in place of the video signal switching unit 42 and that a release image generation unit 55 is added. Note that although a modification of Fig. 2 is applied in an example illustrated in the present embodiment, the circuit regarding the patient information may have the same configuration as in Fig. 1.

The video signal switching unit 52 is provided with a selector 53. The selector 53 includes a switching unit Sd in addition to the switching units Sa to Sc. The 3D image from the image processing unit 22 is provided to one of input ends of the switching unit Sd, and the 2D image from the 2D image generation unit 25 is provided to the other input end. The control unit 28 controls the switching unit Sd, and the switching unit Sd selectively outputs one of two inputs to the release image generation unit 55. The release image generation unit 55 is configured to generate a release image based on an inputted still image when a release signal based on the release operation of the operator is inputted.

A switching signal is provided to the control unit 28, and information based on the switching signal is stored in the memory 28a. The control unit 28 controls each of the switching units Sa to Sc of the selector 53 and the synthesis units 43a to 43c based on the information stored in the memory 28a. As in the modification of Fig. 2, the switching signal is for controlling the switching units Sa to Sc and the synthesis units 43a to 43c.

Furthermore, in the present embodiment, the user can use an input apparatus not shown to perform operation of setting which one of the 3D image and the 2D image is to be recorded at the release operation, and a switching signal based on the operation is also supplied to the control unit 28. The control unit 28 is configured to provide and store, in the memory 28a, the setting information indicating which one of the 3D image and the 2D image is to be recorded at the release operation. The control unit 28 reads the setting information of the memory 28a to control the switching unit Sd to supply the 3D image or the 2D image to the release image generation unit 55 based on the setting information.

The video signal switching unit 52 is also provided with a freeze detection unit 54. A freeze instruction signal based on user operation is provided to the freeze detection unit 54. The freeze detection unit 54 is configured to output a detection result indicating generation of a freeze instruction to the control unit 28 when freeze operation is detected based on the freeze instruction signal.

When the setting information indicating that whether the release image to be recorded is the 3D image or 2D image is not recorded in the memory 28a, the control unit 28 may display a menu display for the user to select one of the 3D image and the 2D image. The control unit 28 can generate display data of the menu display to supply the display data to one of the synthesis units 43a to 43c to display the menu display for the selection on a display screen of a monitor connected to one of the output ports 29a to 29c.

Note that the image processing unit 22 and the 2D image generation unit 25 include a memory not shown and are configured to consecutively output 3D still images or 2D still images at detection of a freeze instruction when the freeze instruction is detected.

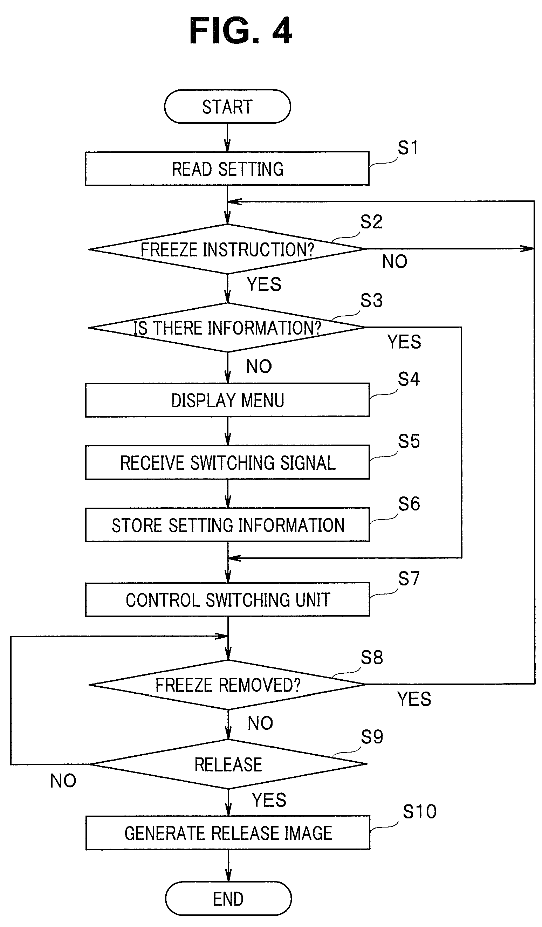

Next, operation of the embodiment with the configuration will be described with reference to a flowchart of Fig. 4.

In step S1 of Fig. 4, the control unit 28 reads the information of the memory 28a. The control unit 28 controls the switching units Sa to Sc and the synthesis units 43a to 43c based on the information stored in the memory 28a. As a result, the output ports 29a to 29c output one of the 3D image and the 2D image separately selected by the user for each output port.

The control unit 28 judges whether the freeze instruction is generated in step S2. Here, it is assumed that the operator operates a freeze switch not shown to instruct freezing. The freeze instruction signal generated by the operation of the freeze switch is supplied to the freeze detection unit 54. When the freeze detection unit 54 detects the freeze operation through the freeze instruction signal, the freeze detection unit 54 outputs this detection result to the control unit 28.

The freeze instruction signal is also provided to the image processing unit 22 and the 2D image generation unit 25. The image processing unit 22 outputs the 3D still image, and the 2D image generation unit 25 outputs the 2D still image. The selector 53 selects one of the images and supplies the image to each of the output ports 29a to 29c. As a result, a 3D or 2D freeze image is provided to the devices connected to the output ports 29a to 29c. The operator can three-dimensionally or two-dimensionally check the freeze image on the monitor connected to one of the output ports 29a to 29c.

The control unit 28 judges whether the information for setting whether to generate the release image by the 3D image or to generate the release image by the 2D image is stored in the memory 28a in step S3. If the information is stored, the control unit 28 moves the process to step S7 and controls the switching unit Sd based on the stored information.

If the setting information is not stored in the memory 28a, the control unit 28 displays the menu display in the following step S4. Display data of the menu display from the control unit 28 is supplied to one of the synthesis units 43a to 43c and displayed by the monitor connected to one of the output ports 29a to 29c. The menu display is for inquiring the user whether to generate the release image by the 3D image or to generate the release image by the 2D image.

The user performs operation for instructing whether to generate the release image by the 3D image or to generate the release image by the 2D image while viewing the menu display. The operation of the user is supplied as a switching signal to the control unit 28 (step S5). The control unit 28 stores the setting information based on the switching signal in the memory 28a (step S6) and controls switching of the switching unit Sd based on the information. As a result, the still image of one of the 3D image and the 2D image selected by the user is supplied to the release image generation unit 55 through the switching unit Sd.

In step S8, whether freeze removal operation was performed is judged. When the freeze removal operation is performed, the image processing unit 22 and the 2D image generation unit 25 stop outputting the still image and restart outputting a movie. As a result, the output ports 29a to 29c output a movie based on 3D images or 2D images.

In step S9, the release operation is judged. When the release operation is performed, the release image generation unit 55 generates a release image based on the image inputted through the switching unit Sd. The switching unit Sd outputs the 3D image or the 2D image according to the setting operation of the user, and the release image generation unit 55 generates a 3D release image or a 2D release image according to the user operation and outputs the release image to a recording medium 56 provided inside of the video processor 55.

In this way, the still image of the 3D image or the 2D image designated by the user can be recorded for the release image in the present embodiment, regardless of whether the image outputted from the output port is a 3D image or a 2D image. As a result, the surgeon can also record the 2D image as the release image while viewing the 3D still image displayed on the monitor by the freeze operation. Therefore, the operator can view the image with a feeling of depth to relatively easily select the image suitable for the release image, and the selected image is recorded as a 2D image with a relatively small amount of data.

Note that although the menu display is displayed to make the user perform the setting only when the setting information indicating whether the release image is to be a 3D image or a 2D image is not stored in the memory 28a in the example illustrated in Fig. 4, the menu display may be displayed before the release operation regardless of whether the setting information is stored to make the user set which one of the 3D image and the 2D image is to be the release image.

Furthermore, although the release operation is not performed unless the freeze operation is not performed in the example described in the example of Fig. 4, the release operation may be performed without the execution of the freeze operation. When the release operation is performed without the involvement of the freeze operation, the still image at the release timing needs to be supplied to the release image generation unit 55. Therefore, in this case, the control unit 28 may cause the switching unit Sd to mandatorily select one of the images, such as the 2D image, when the setting information is not stored in the memory 28a.

### (Another Example)

By the way, when a 3D image is generated from picked-up images for left eye and for right eye outputted by image pickup devices for left eye and for right eye, there may be a difference between the left image and the right image regarding color tone or display position. When there is such a difference, the observed image displayed on the monitor may be unnatural, or image quality may be degraded.

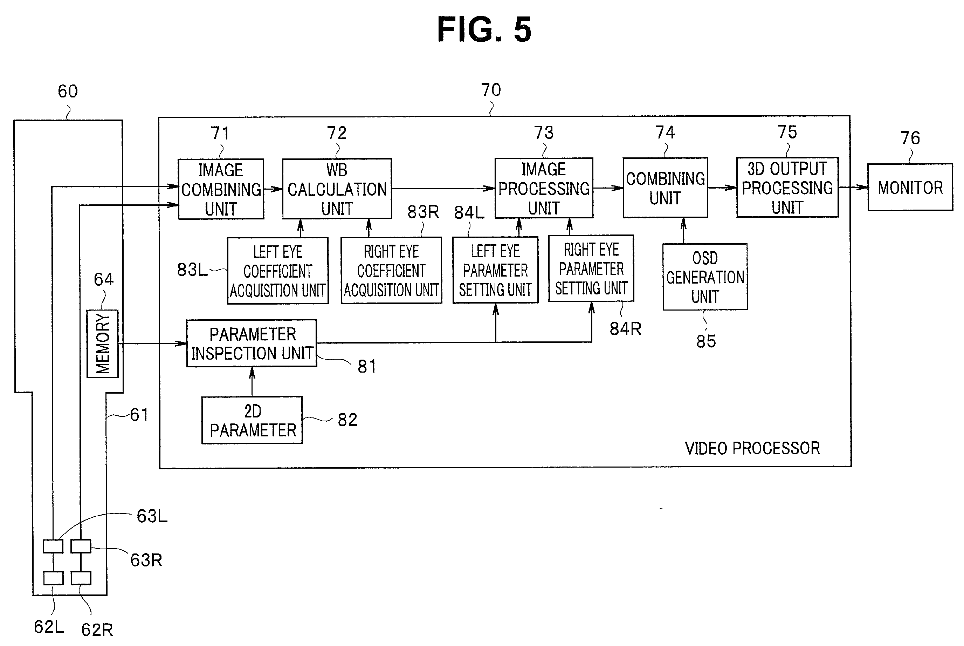

To reduce the difference between the left image and the right image regarding the color tone or the display position, a method of independently setting parameters of image correction for left image and for right image can be implemented. Fig. 5 is a block diagram showing an endoscope system that can independently set the correction parameters of left image and right image.

The endoscope system of Fig. 5 includes an endoscope 60 and a video processor 70. On a distal end side, the endoscope 60 includes an elongated insertion portion 61 that can be inserted to a lumen and the like, and a proximal end side is detachably connected to the video processor 70 through a connector or the like not shown.

An image pickup device 62L for left eye and an image pickup device 62R for right eye that pick up images of a video of an object in a lumen or the like are disposed on a distal end of the insertion portion 61. An illumination lens not shown is disposed on the distal end of the insertion portion 61, and illuminating light is applied to the object through the illumination lens. The image pickup devices 62L and 62R include CCDs, CMOS sensors, or the like, and return light from the object enters each image pickup surface. The image pickup devices 62L and 62R photoelectrically convert entered object optical images and sequentially output image pickup outputs based on accumulated charge.

The picked-up image for left eye from the image pickup device 62L is provided to an A/D converter 63L, and the picked-up image for right eye from the image pickup device 62R is provided to an A/D converter 63R. The A/D converters 63L and 63R convert the inputted picked-up image for left eye and picked-up image for right eye into digital signals, respectively, and output the digital signals to the video processor 70.

The endoscope 60 is also provided with a memory 64. Correction parameters for left eye and correction parameters for right eye for appropriately correcting the left image and the right image according to characteristics of the image pickup devices 62L and 62R are stored in the memory 64. When the endoscope 60 is mounted, the video processor 70 can read the correction parameters for right eye and for left eye stored in the memory 64.

The picked-up images for left eye and for right eye from the endoscope 60 are supplied to an image synthesis unit 71 of the video processor 70. The image synthesis unit 71 synthesizes the picked-up images for left eye and for right eye from the endoscope 60 and outputs a 3D image to a WB calculation unit 72. The WB calculation unit 72 is provided with white balance (WB) coefficients of the left image from a left eye coefficient acquisition unit 83L and performs calculation for white balance adjustment regarding the left image. The WB calculation unit 72 is also provided with white balance (WB) coefficients of the right image from a right eye coefficient acquisition unit 83R and performs calculation for white balance adjustment regarding the right image. Note that the left eye coefficient acquisition unit 83L and the right eye coefficient acquisition unit 83R may independently and simultaneously acquire the WB coefficients.

The 3D image from the WB calculation unit 72 is supplied to an image processing unit 73. The image processing unit 73 applies predetermined image processing to the inputted 3D image and outputs the 3D image to a synthesis unit 74. Examples of the image processing by the image processing unit 73 includes distortion correction, brightness correction, shading correction, rotation correction, size correction, position correction, OB correction, and enhancement processing.

In an example of Fig. 5, the image processing unit 73 is provided with the parameters for left image from a left eye parameter setting unit 84L and applies various corrections to the left image using the parameters for left eye. The image processing unit 73 is provided with the parameters for right image from a right eye parameter setting unit 84R and applies various corrections to the right image using the parameters for right eye.

In the example of Fig. 5, the correction parameters for left eye and for right eye read from the memory 64 of the endoscope 60 are set in the left eye parameter setting unit 84L and the right eye parameter setting unit 84R. The parameter inspection unit 81 inspects the correction parameters for left eye and for right eye read from the memory 64, and if there is no abnormality in the parameters, the parameter inspection unit 81 is configured to provide the read correction parameters without change to the left eye parameter setting unit 84L and the right eye parameter setting unit 84R.

The image processing unit 73 uses the correction parameters for left eye and for right eye stored in the memory 64 of the endoscope 60 to correct the left image and the left image and can make corrections according to the characteristics of the image pickup devices 62L and 62R. The image processing unit 73 uses the correction parameters for left eye and for right eye to independently correct the left image and the left image and can prevent generation of a difference between the left image and the right image regarding the color tone or the display position.

When a parameter inspection unit 81 determines that there is an abnormality in the correction parameters read from the memory 64, the parameter inspection unit 81 reads 2D parameters from a memory 82 and outputs the 2D parameters. As a result, the 2D parameters are provided to the left eye parameter setting unit 84L and the right eye parameter setting unit 84R, and the 2D parameters are used to similarly correct the left and right images.

The 3D image from the image processing unit 73 is provided to the synthesis unit 74. The synthesis unit 74 is provided with display data from an OSD generation unit 85. The synthesis unit 74 synthesizes a display image generated by the OSD generation unit 85 with the endoscopic 3D image and outputs the image to a 3D output processing unit 75. The 3D output processing unit 75 outputs the inputted image to a monitor 76 to project the image on a display screen of the monitor 76. As a result, a 3D image with a sufficiently small difference between left and right images regarding the color tone or the display position is displayed on the display screen of the monitor 76.

Note that when the parameter inspection unit 81 outputs the 2D parameters, the image processing unit 73 may output the 3D image without making corrections, and the 3D output processing unit 75 may output the 2D image instead of the 3D image.

In this way, according to the example of Fig. 5, the left eye coefficient acquisition unit 83L and the right eye coefficient acquisition unit 83R independently and simultaneously acquire the white balance coefficients. As a result, a time period required for the acquisition of the left and right white balance coefficients can be reduced. The WB calculation unit 72 is configured to independently multiply the left and right images by the WB coefficients and can adjust the white balance according to the characteristics of the left and right image pickup devices 62L and 62R. Furthermore, the image processing unit 73 uses the correction parameters read from the endoscope 60 to independently correct the left and right images and can execute image processing according to the characteristics of the left and right image pickup devices 62L and 62R. Therefore, in the example of Fig. 5, the left and the right can be independently corrected regarding the color tone or the display position, and the difference between the left and right images can be reduced to improve the image quality regardless of the difference in the characteristics or the like of the left and right image pickup devices.

The present invention is not limited to the embodiments, and in the execution phase, the present invention can be embodied by modifying the constituent elements without departing from the scope of the present invention. In addition, various inventions can be formed by appropriate combinations of a plurality of constituent elements disclosed in each of the embodiments. For example, some of the constituent elements illustrated in the embodiments may be deleted. Furthermore, the constituent elements across different embodiments may be appropriately combined.

The present application is filed on the basis of claiming the benefit of priority from Japanese Patent Application No. 2013-217376, filed on October 18, 2013 in Japan, and the disclosed contents are incorporated in the present specification, the claims, and the drawings by reference.

## Claims

1. An image output apparatus comprising:
a stereoscopic image generation unit that is provided with a first image and a second image with a parallax relative to the first image and that generates a stereoscopic image based on the first image and the second image, wherein the first image is provided from a first image acquisition unit that acquires the first image by picking up an image of a subject, and the second image is provided from a second image acquisition unit that acquires the second image by picking up an image of the subj ect;
a two-dimensional image generation unit that generates a two-dimensional image based on the first image or the second image;
a plurality of output units that can output the stereoscopic image and the two-dimensional image; and
a switching unit that can switch, for each of the output units, which image of the stereoscopic image and the two-dimensional image is supplied to the plurality of output units.

2. The image output apparatus according to claim 1, wherein
in the switching unit, the switching of each of the output units is controlled based on instruction operation of a user or information stored in a memory.

3. The image output apparatus according to claim 1 or 2, comprising:
a patient information acquisition unit that acquires patient information; and
a synthesis unit that patient information that can switch, for each of the output units, whether to synthesize the patient information acquired by the patient information acquisition unit with the stereoscopic image and the two-dimensional image supplied to the output units.

4. The image output apparatus according to any one of claims 1 to 3, wherein
the switching unit can switch which image of the stereoscopic image and the two-dimensional image is provided to a recording unit to cause the recording unit to record the image.

5. The image output apparatus according to any one of claims 1 to 4, wherein in the switching unit, the switching is controlled based on instruction operation of a user or information stored in a memory.

6. The image output apparatus according to any one of claims 1 to 5, comprising:
a recording unit that is provided with a still image of the stereoscopic image or a still image of the two-dimensional image and that records the still image.

7. The image output apparatus according to claim 6, comprising:
a still image acquisition unit that acquires the still image of the stereoscopic image or the still image of the two-dimensional image at timing based on freeze operation of a user and that records the still image in the recording unit, wherein
the switching unit can switch which image of the still image of the stereoscopic image and the still image of the two-dimensional image is acquired by the still image acquisition unit regardless of switching control regarding to which output unit the stereoscopic image and the two-dimensional image are supplied.

8. The image output apparatus according to claim 7, wherein
the switching unit switches which image of the still image of the stereoscopic image and the still image of the two-dimensional image is acquired by the still image acquisition unit based on instruction operation of the user or information stored in the memory.

9. The image output apparatus according to claim 7, wherein
the switching unit displays a display for receiving user operation for switching which image of the still image of the stereoscopic image and the still image of the two-dimensional image is acquired by the still image acquisition unit.
